(19) Europäisches Patentamt
European Patent Office
Office européen des brevets

(11) EP 2 958 630 B1

(12) **EUROPEAN PATENT SPECIFICATION**

(45) Date of publication and mention of the grant of the patent:
**27.03.2019 Bulletin 2019/13**

(21) Application number: **14708694.6**

(22) Date of filing: **25.02.2014**

(51) Int Cl.:
***A61Q 1/02*** (2006.01) ***A61Q 19/00*** (2006.01)
***A61K 8/81*** (2006.01) ***A61K 8/88*** (2006.01)
***A61K 8/04*** (2006.01) ***A61Q 1/06*** (2006.01)
***A61Q 1/10*** (2006.01) ***A61Q 1/12*** (2006.01)
***A61K 8/84*** (2006.01) ***A61K 8/89*** (2006.01)
***A61K 8/898*** (2006.01) ***A61K 8/891*** (2006.01)

(86) International application number:
**PCT/IB2014/059238**

(87) International publication number:
**WO 2014/128678 (28.08.2014 Gazette 2014/35)**

# (54) GEL-TYPE COSMETIC COMPOSITION

GELFÖRMIGE KOSMETISCHE ZUSAMMENSETZUNG

COMPOSITION COSMETIQUE DE TYPE GEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.02.2013 FR 1300429**

(43) Date of publication of application:
**30.12.2015 Bulletin 2015/53**

(73) Proprietor: **L'Oréal**
**75008 Paris (FR)**

(72) Inventors:
- **FERRARI, Véronique**
**F-94700 Maisons-Alfort (FR)**
- **VALVERDE, Elodie**
**F-75012 Paris (FR)**

(74) Representative: **Nony**
**11 rue Saint-Georges**
**75009 Paris (FR)**

(56) References cited:
**WO-A1-99/62497**
**WO-A1-99/65455**
**WO-A2-2008/081175**

- **DATABASE WPI Week 198508 Thomson Scientific, London, GB; AN 1985-047704 XP002716202, & JP S60 6607 A (POLA KASEI KOGYO KK) 14 January 1985 (1985-01-14)**
- **ALMEIDA I F ET AL: "Evaluation of the physical stability of two oleogels", INTERNATIONAL JOURNAL OF PHARMACEUTICS, ELSEVIER BV, NL, vol. 327, no. 1-2, 11 December 2006 (2006-12-11), pages 73-77, XP027972479, ISSN: 0378-5173 [retrieved on 2006-12-11]**
- **ALMEIDA,FERNANDES,PENA FERREIRA,COSTA,BAHIA: "Moisturizing effect of oleogel/hydrogel mixtures", PHARMACEUTICAL DEVELOPMENT AND TECHNOLOGY, vol. 13, 2 January 2008 (2008-01-02), pages 487-494, XP009174126, ISSN: 1083-7450**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention is directed towards proposing for the field of caring for and making up keratin materials, especially the skin and/or the lips, and in particular the skin, a novel galenical form that is most particularly advantageous with regard to its technical performance and the sensations it affords the user during its application, in particular to the skin.

The term *"keratin materials"* especially means the skin, the lips and/or the eyelashes, in particular the skin and/or the lips, and preferably the skin.

Conventionally, a cosmetic composition formulator uses emulsified systems combining an aqueous phase for freshness and an oily phase for comfort. The strong point of these systems is also that they allow the combination, within the same composition, of cosmetic ingredients or active agents that have different affinities with respect to these two aqueous and oily phases.

Unfortunately, these emulsified systems do not lend themselves to rapid and easy production of an infinite range of compositions. Thus, for a given emulsified system, it often proves complicated to functionalize the formulation by adding, for example, an antisun product, certain active agents, pigments, polymers, fragrances or fillers, etc. without impairing the stability, the sensory properties and the quality of the film deposited on the keratin materials and especially the skin. The formulation then needs to be readjusted. It is also difficult to reconcile, within the same composition, opposing technical performance qualities, for instance mattness (which may make the skin dry) and moisturization (which may make the skin shiny).

Furthermore, emulsified systems do not lend themselves to the formulation of all the ingredients or active agents liable to be considered in the field of care and/or makeup, or even to the formulation of high contents of certain cosmetic ingredients or active agents. Non-compliance with these incompatibilities has the consequence of destabilizing the emulsified architecture, which then, inter alia, undergoes demixing.

Finally, these emulsified systems do not lend themselves to rapid and easy production of an infinite range of textures.

Moreover, in the case of making up the complexion, the preferred emulsifying systems are mainly reverse emulsions with regard to the good level of coverage and the homogeneous appearance they afford when compared with direct emulsions. On the other hand, their weak point is a high greasy and tacky sensation, and thus a lack of lightness as regards the textures obtained.

Galenical formulations of gel/gel type partially meet these expectations (Almeida et al., Pharmaceutical Development and Technology, 2008, 13:487, tables 1 and 2, page 488; WO 99/65455; PI 0405758-9; WO 99/62497; JP 2005-112834 and WO 2008/081175). Formulations of this type combine a gelled aqueous phase with a gelled oily phase. In fact, these gel/gel formulations were essentially proposed as an advantageous alternative to emulsified systems on the grounds that they make it possible to dispense with the use of the surfactants required for the stability and texturization of emulsions. Unfortunately, besides this advantage, the gel/gel formulations described hitherto do not essentially reveal any novel or improved technical performance qualities.

It therefore remains difficult for a person skilled in the art to propose homogeneous compositions that are capable of affording an immediate visual result on the skin with a light sensation on application, this expected immediate result preferentially being good coverage of colour imperfections and/or of relief imperfections, without, however, marking them. It is therefore necessary to find novel systems for distributing on the skin components such as water, fatty substances and solid particles.

These novel architectures must be entirely satisfactory to users as regards the sensation afforded, but must also be capable of affording improved cosmetic properties, or must even have an increased number of technical performance qualities such as freshness, lightness, emollience, comfort, coverage of imperfections, colour, unifying aspect, lightening, etc., and, on the other hand, must be free of the known side effects of oily and aqueous phases such as, respectively, a greasy feel, a tacky feel, a feeling of lack of glidance or alternatively a feeling of dragging on application.

The inventors have now found, unexpectedly, that such an objective can be achieved via the choice of a system of specific hydrophilic gelling agent(s)/lipophilic gelling agent(s) for the preparation of a cosmetic composition of the type such as a bi-continuous but on the other hand macroscopically homogeneous system which has a large number of technical performance qualities and which furthermore has optimized effects.

More precisely, the inventors have found that the choice of a system of specific hydrophilic gelling agent(s)/lipophilic gelling agent(s) makes it possible, contrary to all expectation, to combine in a single composition a significant number of technical performance qualities, with the intensity of each performance quality advantageously not being attenuated by the manifestation of other associated performance qualities, or even being, for certain performance qualities, stimulated.

Thus, the present application describes a cosmetic composition for making up and/or caring for keratin materials, in particular the skin and/or the lips, comprising:

- at least one aqueous phase gelled with at least one hydrophilic gelling agent; and

- at least one oily phase gelled with at least one hydrogen bonding polymer;

the said phases forming therein a macroscopically homogeneous mixture.

According to one of its aspects, the present invention relates to a cosmetic composition for making up and/or caring for keratin materials, in particular the skin and/or the lips, comprising:

- at least one aqueous phase gelled with at least one hydrophilic gelling agent chosen from synthetic polymeric gelling agents; and
- at least one oily phase gelled with at least one hydrogen bonding polymer chosen from hydrocarbon-based polyamides and silicone polyamides, and mixtures thereof;

the said phases forming therein a macroscopically homogeneous mixture,
wherein the composition contains, as hydrophilic gelling agent(s)/lipophilic gelling agent(s) system, a system chosen from:

- copolymer of 2-acrylamido-2-methylpropanesulfonic acid and of hydroxyethyl acrylate/hydrocarbon-based polyamide; and
- copolymer of 2-acrylamido-2-methylpropanesulfonic acid and of hydroxyethyl acrylate/silicone polyamide

According to one embodiment variant, a composition according to the invention consists of an aqueous phase gelled with at least one hydrophilic gelling agent and an oily phase gelled with at least one hydrogen bonding polymer.

According to a preferred variant, a composition according to the invention also contains at least one dyestuff.

This dyestuff may be chosen from pigments, and water-soluble or liposoluble dyestuffs, especially as detailed below.

In particular, the dyestuffs are pigments.

According to an advantageous embodiment variant, the dyestuff is conveyed at least in the gelled oily phase.

As stated above, the inventors have found, contrary to all expectation, that the choice of particular hydrophilic gelling agent(s)/lipophilic gelling agent(s) couples for texturing a composition of gel/gel type makes it possible to significantly improve certain technical performance qualities, and to dispense with certain adverse effects inherent in the gelling agents under consideration, or even to reconcile within this composition properties which it was hitherto difficult to make coexist. Furthermore, as emerges from the examples below, the present invention moreover makes it possible, unexpectedly, to optimize some of the expected technical performance qualities.

The inventors have also found, surprisingly, that the soft-focus performance quality of a composition according to the invention comprising aqueous and oily phases gelled, respectively, with a polymeric or particulate gelling agent with a soft-focus effect proves to be significantly improved. The gain in soft-focus effect proves to be greater than the sum of the respective optical effects of each of the two gelled phases in each of the two compositions. There is manifestly synergism.

Besides the abovementioned unexpected advantages, the gelling system under consideration according to the invention affords a texture that is sufficiently thickened to be compatible with the formulation of a very wide diversity of ingredients or active agents. It combines in a single formulation a large number of functional active agents or ingredients (fillers, pigments, etc.).

The compositions according to the invention also prove to be very stable and not subject to syneresis.

The present application also describes a process for preparing a cosmetic composition for making up and/or caring for keratin materials, in particular the skin and/or the lips, comprising at least one step of mixing:

- at least one aqueous phase gelled with at least one hydrophilic gelling agent; and
- at least one oily phase gelled with at least one hydrogen bonding polymer;

under conditions suitable for obtaining a macroscopically homogeneous mixture.

According to one of its aspects, the present invention relates to a process for preparing a cosmetic composition for making up and/or caring for keratin materials, in particular the skin and/or the lips, comprising at least one step of mixing:

- at least one aqueous phase gelled with at least one hydrophilic gelling agent; and
- at least one oily phase gelled with at least one hydrogen bonding polymer;

under conditions suitable for obtaining a macroscopically homogeneous mixture the hydrophilic gelling agent(s)/lipophilic gelling agent(s) system being chosen from:

- copolymer of 2-acrylamido-2-methylpropanesulfonic acid and of hydroxyethyl acrylate/hydrocarbon-based polyamide; and

- copolymer of 2-acrylamido-2-methylpropanesulfonic acid and of hydroxyethyl acrylate/silicone polyamide.

According to one embodiment variant, this process may advantageously comprise a step of mixing at least three or even more gelled phases.

For obvious reasons, the number of gelled aqueous phases and of gelled oily phases to be considered for forming a composition according to the invention may range for each of the two types of phase beyond two. It is especially conditioned by the number of expected technical performance qualities.

For example, this process may use a single gelled aqueous phase and two oily phases gelled with different lipophilic gelling agents.

Conversely, this process may also use a single gelled oily phase and two aqueous phases gelled with different hydrophilic gelling agents.

For example, the phases having the same architecture, namely aqueous or oily, may be precombined to form a premix, and it is this premix which is placed in contact with the phase or even with a premix of several phases having the other architecture.

The corresponding aqueous and oily gels may be prepared separately then mixed together without heating, without requiring the necessary presence of surfactants in order to achieve the desired architecture. Thus, in addition to the advantages mentioned above, the claimed compositions may be readily prepared at reduced cost.

Advantageously, the mixing of the phases may be performed at room temperature.

However, the process of the invention may comprise, if necessary, a step of heating the mixture.

The process according to the invention thus offers the formulator a simple and rapid means for gaining access to a multitude of cosmetic compositions having common performance qualities but also performance qualities that are specific to each of its compositions.

The present invention also gives the user access to this faculty of mixing at least two phases of the same architecture with at least one phase of different architecture *via* the provision of a cosmetic kit for making up and/or caring for keratin materials.

Thus, the present application describes a cosmetic kit for making up and/or caring for keratin materials, in particular the skin and/or the lips, comprising, in separate containers, at least one aqueous phase gelled with at least one hydrophilic gelling agent; and at least one oily phase gelled with at least one hydrogen bonding polymer; and also to instructions for using the extemporaneous mixtures.

According to one of its aspects, the present invention relates to a cosmetic kit for making up and/or caring for keratin materials, in particular the skin and/or the lips, comprising, in separate containers, at least one aqueous phase gelled with at least one hydrophilic gelling agent, and at least one oily phase gelled with at least one hydrogen bonding polymer, and also instructions for using the extemporaneous mixtures, the hydrophilic gelling agent(s)/lipophilic gelling agent(s) system being chosen from:

- copolymer of 2-acrylamido-2-methylpropanesulfonic acid and of hydroxyethyl acrylate/hydrocarbon-based polyamide; and
- copolymer of 2-acrylamido-2-methylpropanesulfonic acid and of hydroxyethyl acrylate/silicone polyamide.

The present application describes a device for making up and/or caring for keratin materials, in particular the skin and/or the lips, comprising at least:

- two separate containers containing, respectively, at least one aqueous phase gelled with at least one hydrophilic gelling agent and at least one oily phase gelled with at least one hydrogen bonding polymer;
- a distinct chamber for mixing the said containers, comprising an aperture configured to allow the introduction of the said phases to be mixed; and
- a means for distributing a macroscopically homogeneous mixture of the two phases.

According to one of its aspects, the present invention relates to a device for making up and/or caring for keratin materials, in particular the skin and/or the lips, comprising at least:

- two separate containers containing, respectively, an aqueous phase gelled with at least one hydrophilic gelling agent, and an oily phase gelled with at least one hydrogen bonding polymer, the hydrophilic gelling agent(s)/lipophilic gelling agent(s) system being chosen from:
- copolymer of 2-acrylamido-2-methylpropanesulfonic acid and of hydroxyethyl acrylate/hydrocarbon-based polyamide; and
- copolymer of 2-acrylamido-2-methylpropanesulfonic acid and of hydroxyethyl acrylate/silicone polyamide;

- a distinct chamber for mixing the said containers, comprising an aperture configured to allow the introduction of the said phases to be mixed; and
- a means for distributing a macroscopically homogeneous mixture of the two phases.

According to an advantageous variant, the kits and devices according to the invention contain at least two, or even more, different gelled phases for each of the two types of aqueous and oily architecture.
According to a particular embodiment, the representative gelled phases of the same type of architecture are gelled with a different gelling agent.
Multi-phase formulations of *"patchwork"* type may thus be developed.
According to another particular embodiment, the representative gelled phases of the same type of architecture are different as regards their optical properties. For example, the kit or device may propose two oily gelled phases textured by the same oily gelling agent, but one containing dyestuffs and the other not. The user thus has the possibility of exploiting or not exploiting makeup performance quality in addition to the other performance qualities.
A kit or device according to the invention also allows the user to modify the intensity of the colour effect by adjusting the proportion of the coloured gelled phase to be mixed.
Thus, the kits and devices according to the invention are particularly advantageous in so far as they afford the user the possibility of adjusting at will, by means of the choice of the gelled phases representative of the two types of oily and aqueous architecture, the desired makeup performance qualities, while at the same time ensuring convenience and ease of use.
The present invention especially makes it possible to afford the user a wider makeup range and also to give the makeup operation an appealing fun aspect. Moreover, the fact that the mixing of the phases may be performed at room temperature is of manifest interest as regards the convenience and thus gives satisfaction as regards the simplicity of use.
According to another of its aspects, a subject of the invention is also a process, especially a cosmetic process, for making up and/or caring for a keratin material, in particular the skin and/or the lips, comprising at least one step that consists in applying to the said keratin material a composition in accordance with the invention.
The present application describes a process, especially a cosmetic process, for caring for and/or making up a keratin material, in particular the skin and/or the lips, comprising at least the application to the said material of a composition, in particular a macroscopically homogeneous composition obtained by extemporaneous mixing, before application or at the time of application to the said keratin material, of at least one aqueous phase gelled with at least one hydrophilic gelling agent, and of at least one oily phase gelled with at least one hydrogen bonding polymer.
According to one of its aspects, the present invention relates to a cosmetic process for making up and/or caring for a keratin material, in particular the skin and/or the lips, comprising at least the application to the said material of a composition obtained by extemporaneous mixing, before application or at the time of application to the said keratin material, of at least one aqueous phase gelled with at least one hydrophilic gelling agent, and at least one oily phase gelled with at least one hydrogen bonding polymer, said composition containing, as hydrophilic gelling agent(s)/lipophilic gelling agent(s) system, a system chosen from:

- copolymer of 2-acrylamido-2-methylpropanesulfonic acid and of hydroxyethyl acrylate/hydrocarbon-based polyamide; and
- copolymer of 2-acrylamido-2-methylpropanesulfonic acid and of hydroxyethyl acrylate/silicone polyamide.

## COSMETIC COMPOSITION

Firstly, it is important to note that a composition according to the invention is different from an emulsion.
An emulsion generally consists of an oily liquid phase and an aqueous liquid phase. It is a dispersion of droplets of one of the two liquid phases in the other. The size of the droplets forming the dispersed phase of the emulsion is typically about a micrometer (0.1 to 100 $\mu$m). Furthermore, an emulsion requires the presence of a surfactant or of a silicone emulsifier to ensure its stability over time.
In contrast, a composition according to the invention consists of a macroscopically homogeneous mixture of two immiscible gelled phases. These two phases both have a gel-type texture. This texture is especially reflected visually by a consistent, creamy appearance.
The term *"macroscopically homogeneous mixture"* means a mixture in which each of the gelled phases cannot be individualized with the naked eye.
More precisely, in a composition according to the invention, the gelled aqueous phase and the gelled oily phase interpenetrate and thus form a stable, and consistent product. This consistency is achieved by mixing interpenetrated oily and aqueous gelled macrodomains. These interpenetrated macrodomains are not measurable objects. Thus, by microscope, the composition according to the invention is very different from an emulsion. It cannot be characterized

either as having a *"sense",* i.e. an O/W or W/O sense.

Thus, a composition according to the invention has a gel-type consistency. Furthermore, the stability of the composition is long-lasting without surfactant. Consequently, a cosmetic composition according to the invention does not require any surfactant or silicone emulsifier to ensure its stability over time.

It is known from the state of the art to observe the intimate nature of the mixture of the aqueous and oily gels in a gel-type composition, for example, by introducing a dye substance into either the oily or aqueous gel phases before forming the gel-type composition. On visual inspection, the dye is seen to be uniformly dispersed, even though the dye is present in only one of the oily gel or aqueous gel. Indeed, if two different dyes of different colours are introduced into the oily and aqueous phases, respectively, before forming the gel-type composition, both colours can be observed uniformly dispersed throughout the gel-type composition. This is in contrast to an emulsion wherein if a dye that is either water-soluble or oil-soluble is introduced into the aqueous or oily phases, respectively, before forming an emulsion, only the colour of the dye in the external phase will be observed (Remington: The Science and Practice of Pharmacy, 19th Edition (1995) Chapter 21, page 282).

It is also known to distinguish a gel-type composition from an emulsion by performing a "drop test". This test consists to demonstrate the bi-continous nature of a gel-type composition. Indeed, as mentioned above, the composition’s consistency is achieved by interpenetrating oily and aqueous gelled domains. Therefore, the bi-continous nature of a gel-type composition can be highlighted by a simple test with respectively hydrophilic and hydrophobic solvents. This test consists to deposit, on the one hand, a droplet of a hydrophilic solvent on a first sample of the tested composition, and, on the other hand, a droplet of hydrophobic solvent on a second sample of the same tested composition, and to analyze the behavior of both droplets of solvents. In the case of an O/W emulsion, a droplet of hydrophilic solvent diffuses in the sample and a droplet of hydrophobic solvent remains at the sample surface. In the case of a W/O emulsion, a droplet of hydrophilic solvent remains at the sample surface and a droplet of hydrophobic solvent diffuses throughout sample. Finally, in the case of a gel-type composition (bi-continuous system), the hydrophilic and hydrophobic droplets diffuse in the entire sample.

In particular, in the case of the present invention, the test which will be privileged for distinguishing a gel-type composition from an emulsion consists in a dilution test. Indeed, in a gel-type composition, the gelled aqueous domains and gel oily domains interpenetrate and form a stable and consistent product, whose dilution behavior in water and oil is different of emulsion’s behavior. Therefore, the dilution behavior of a gel-type composition (bi-continuous system) can be compared to emulsions.

More specifically, the dilution test consists to put 40g of product plus 160g of dilution solvent (water or oil) in a 30 ml plastic beaker. The dilution is performed under controlled agitation to avoid any phenomenon of emulsification. In particular, it is done using a planetary mixer: Speed Mixer TM DAC400FVZ. The Speed Mixer is set to 1500 rpm for 4 minutes. Finally, observation of resulting sample is made with a light microscope at a magnification of x 100 (x10x10). It may be noticed that oils like Parleam® and Xiameter PMX-200 Silicone Fluid 5CS® from Dow Corning are convenient as dilution solvents.

In the case of a gel-type composition (bi-continuous system), when diluted either in oil or water, a heterogeneous aspect is always observed. When a gel-type composition (bi-continuous system) is diluted with water, one will observe lumps of oily gel in suspension and when a gel-type composition (bi-continuous system) is diluted with oil, one will observe lumps of aqueous gel in suspension.

On the contrary, upon dilution, emulsions display a different behavior. An O/W emulsion when it is diluted with an aqueous solvent will gradually thin up without presenting a heterogeneous and lumpy aspect. This same O/W emulsion when diluted with oil will present a heterogeneous appearance (lumps of O/W emulsion suspended in oil). A W/O emulsion when diluted with an aqueous solvent will present a heterogeneous appearance (lumps of W/O emulsion is suspended in the water). This same W/O emulsion when diluted with oil will gradually thin up without presenting a heterogeneous and lumpy aspect.

In general, the aqueous gelled phase and the oily gelled phase forming a composition according to the invention are present therein in a weight ratio ranging from 95/5 to 5/95. More preferentially, the aqueous phase and the oily phase are present in a weight ratio ranging from 30/70 to 80/20.

The ratio between the two gelled phases is adjusted according to the desired cosmetic properties.

Thus, in the case of a composition intended for making up the skin and especially the face, it is advantageous to favour an aqueous phase/oily phase weight ratio greater than 1, especially ranging from 60/40 to 90/10, preferably ranging from 60/40 to 80/20, preferably from 60/40 to 70/30, and more preferably to favour an aqueous phase/oily phase weight ratio of 60/40 or 70/30.

These preferred ratios are particularly advantageous for obtaining fresh and light compositions.

Advantageously, a composition according to the invention is in the form of a creamy gel with a minimum stress below which it does not flow unless it has been subjected to an external mechanical stress.

As emerges from the text hereinbelow, a composition according to the invention may have a minimum threshold stress of 1.5 Pa and in particular greater than 10 Pa.

It may also advantageously have a stiffness modulus G* at least equal to 400 Pa and preferably greater than 1000 Pa.

According to an advantageous embodiment variant, the gelled phases under consideration to form a composition according to the invention may have, respectively, a threshold stress of greater than 1.5 Pa and preferably greater than 10 Pa.

Characterization of the threshold stresses is performed by oscillating rheology measurements. A method is proposed in the examples section of the present text.

In general, the corresponding measurements are taken at 25°C using a Haake RS600 imposed-stress rheometer equipped with a plate-plate measuring body (60 mm diameter) fitted with an anti-evaporation device (bell jar). For each measurement, the sample is placed delicately in position and the measurements start 5 minutes after placing the sample in the air gap (2 mm). The tested composition is then subjected to a stress ramp from $10^{-2}$ to $10^{3}$ Pa at a set frequency of 1 Hz.

A composition according to the invention may also have a certain elasticity. This elasticity may be characterized by a stiffness modulus G* which, under this minimum stress threshold, may be at least equal to 400 Pa and preferably greater than 1000 Pa. The value G* of a composition may be obtained by subjecting the composition under consideration to a stress ramp from $10^{-2}$ to $10^{3}$ Pa at a set frequency of 1 Hz.

## HYDROPHILIC GELLING AGENT

For the purposes of the present invention, the term *"hydrophilic gelling agent"* means a compound that is capable of gelling the aqueous phase of the compositions according to the invention.

The gelling agent is hydrophilic and is thus present in the aqueous phase of the composition.

The gelling agent may be water-soluble or water-dispersible.

As stated above, the aqueous phase of a composition according to the invention is gelled with at least one hydrophilic gelling agent.

The hydrophilic gelling agent is chosen from synthetic polymeric gelling agents.

### Synthetic polymeric gelling agents

A hydrophilic gelling agent according to the invention is at least one synthetic polymeric gelling agent chosen from crosslinked and/or neutralized 2-acrylamido-2-methylpropanesulfonic acid copolymers.

For the purposes of the invention, the term *"synthetic"* means that the polymer is neither naturally existing nor a derivative of a polymer of natural origin.

### Polyacrylamides and crosslinked and/or neutralized 2-acrylamido-2-methylpropanesulfonic acid polymers and copolymers

The hydrophilic gelling agents according to the invention are chosen from synthetic polymeric gelling agents chosen from copolymer of 2-acrylamido-2-methylpropanesulfonic acid and of hydroxyethyl acrylate.

Polymers suitable as aqueous gelling agent may be crosslinked or non-crosslinked homopolymers or copolymers comprising at least the 2-acrylamidomethylpropanesulfonic acid (AMPS®) monomer, in a form partially or totally neutralized with a mineral base other than aqueous ammonia, such as sodium hydroxide or potassium hydroxide.

They are preferably totally or almost totally neutralized, i.e. at least 90% neutralized.

These AMPS® polymers may be crosslinked or non-crosslinked.

When the polymers are crosslinked, the crosslinking agents may be chosen from the polyolefinically unsaturated compounds commonly used for crosslinking polymers obtained by free-radical polymerization.

Examples of crosslinking agents that may be mentioned include divinylbenzene, diallyl ether, dipropylene glycol diallyl ether, polyglycol diallyl ethers, triethylene glycol divinyl ether, hydroquinone diallyl ether, ethylene glycol or tetraethylene glycol di(meth)acrylate, trimethylolpropane triacrylate, methylenebisacrylamide, methylenebismethacrylamide, triallylamine, triallyl cyanurate, diallyl maleate, tetraallylethylenediamine, tetraallyloxyethane, trimethylolpropane diallyl ether, allyl (meth)acrylate, allylic ethers of alcohols of the sugar series, or other allyl or vinyl ethers of polyfunctional alcohols, and also the allylic esters of phosphoric and/or vinylphosphonic acid derivatives, or mixtures of these compounds.

The crosslinking agent may be chosen from methylenebisacrylamide, allyl methacrylate and trimethylolpropane triacrylate (TMPTA). The degree of crosslinking generally ranges from 0.01 mol% to 10 mol% and more particularly from 0.2 mol% to 2 mol% relative to the polymer.

The AMPS® polymers are water-soluble or water-dispersible. They are in this case:

- either "homopolymers" comprising only AMPS monomers and, if they are crosslinked, one or more crosslinking agents such as those defined above;
- or copolymers obtained from AMPS® and from one or more hydrophilic or hydrophobic ethylenically unsaturated monomers and, if they are crosslinked, one or more crosslinking agents such as those defined above. When said copolymers comprise hydrophobic ethylenically unsaturated monomers, the latter do not comprise a fatty chain and are preferably present in small amounts.

For the purpose of the present invention, the term *"fatty chain"* is intended to mean any hydrocarbon-based chain containing at least 7 carbon atoms.

The term *"water-soluble or water-dispersible"* means polymers which, when introduced into an aqueous phase at 25°C, at a mass concentration equal to 1%, make it possible to obtain a macroscopically homogeneous and transparent solution, i.e. a solution with a light maximum transmittance value, at a wavelength equal to 500 nm, through a sample 1 cm thick, of at least 60% and preferably of at least 70%.

The *"homopolymers"* are preferably crosslinked and neutralized, and they may be obtained according to the preparation process comprising the following steps:

(a) the monomer such as AMPS in free form is dispersed or dissolved in a solution of tert-butanol or of water and tert-butanol;
(b) the monomer solution or dispersion obtained in (a) is neutralized with one or more mineral or organic bases, preferably aqueous ammonia $NH_3$, in an amount making it possible to obtain a degree of neutralization of the sulfonic acid functions of the polymer ranging from 90% to 100%;
(c) the crosslinking monomer(s) are added to the solution or dispersion obtained in (b);
(d) a standard free-radical polymerization is performed in the presence of free-radical initiators at a temperature ranging from 10 to 150°C; the polymer precipitates in the tert-butanol-based solution or dispersion.

The water-soluble or water-dispersible AMPS® copolymers according to the invention contain water-soluble ethylenically unsaturated monomers, hydrophobic monomers, or mixtures thereof.

The water-soluble co-monomers may be ionic or nonionic.

Among the ionic water-soluble co-monomers, mention may be made, for example, of the following compounds and salts thereof:

- (meth)acrylic acid,
- styrenesulfonic acid,
- vinylsulfonic acid and (meth)allylsulfonic acid,
- vinylphosphonic acid,
- maleic acid,
- itaconic acid,
- crotonic acid,
- water-soluble vinyl monomers of formula (A) below:

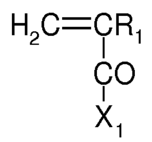

(A)

in which:

- $R_1$ is chosen from H, $-CH_3$, $-C_2H_5$ and $-C_3H_7$;
- $X_1$ is chosen from:
- alkyl oxides of type $-OR_2$ where $R_2$ is a linear or branched, saturated or unsaturated hydrocarbon-based radical containing from 1 to 6 carbon atoms, substituted with at least one sulfonic ($-SO_3-$) and/or sulfate ($-SO_4-$) and/or phosphate ($-PO_4H_2-$) group.

Among the nonionic water-soluble co-monomers, mention may be made, for example, of:

- (meth)acrylamide,
- N-vinylacetamide and N-methyl-N-vinylacetamide,

- N-vinylformamide and N-methyl-N-vinylformamide,
- maleic anhydride,
- vinylamine,
- N-vinyllactams comprising a cyclic alkyl group containing from 4 to 9 carbon atoms, such as N-vinylpyrrolidone, N-butyrolactam and N-vinylcaprolactam,
- vinyl alcohol of formula $CH_2$=CHOH,
- water-soluble vinyl monomers of formula (B) below:

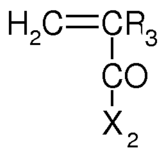

(B)

in which:

- $R_3$ is chosen from H, $-CH_3$, $-C_2H_5$ and $-C_3H_7$;
- $X_2$ is chosen from:
- alkyl oxides of the type $-OR_4$ where $R_4$ is a linear or branched, saturated or unsaturated hydrocarbon-based radical having from 1 to 6 carbon atoms, optionally substituted with a halogen (iodine, bromine, chlorine or fluorine) atom; a hydroxyl (-OH) group; ether.

Mention may be made, for example, of glycidyl (meth)acrylate, hydroxyethyl methacrylate, and (meth)acrylates of ethylene glycol, of diethylene glycol or of polyalkylene glycol.

Among the hydrophobic comonomers without a fatty chain, mention may be made, for example, of:

- styrene and derivatives thereof, such as 4-butylstyrene, $\alpha$-methylstyrene and vinyltoluene;
- vinyl acetate of formula $CH_2$=CH-$OCOCH_3$;
- vinyl ethers of formula $CH_2$=CHOR in which R is a linear or branched, saturated or unsaturated hydrocarbon-based radical containing from 1 to 6 carbons;
- acrylonitrile;
- caprolactone;
- vinyl chloride and vinylidene chloride;
- silicone derivatives, which, after polymerization, result in silicone polymers such as methacryloxypropyltris(trimethylsiloxy)silane and silicone methacrylamides;
- hydrophobic vinyl monomers of formula (C) below:

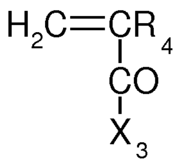

(C)

in which:

- $R_4$ is chosen from H, $-CH_3$, $-C_2H_5$ and $-C_3H_7$;
- $X_3$ is chosen from:
- alkyl oxides of the type $-OR_5$ where $R_5$ is a linear or branched, saturated or unsaturated hydrocarbon-based radical containing from 1 to 6 carbon atoms.

Mention may be made, for example, of methyl methacrylate, ethyl methacrylate, n-butyl (meth)acrylate, tert-butyl (meth)acrylate, cyclohexyl acrylate, isobornyl acrylate and 2-ethylhexyl acrylate.

The water-soluble or water-dispersible AMPS® polymers of the invention preferably have a molar mass ranging from 50 000 g/mol to 10 000 000 g/mol, preferably from 80 000 g/mol to 8 000 000 g/mol, and even more preferably from 100 000 g/mol to 7 000 000 g/mol.

As water-soluble or water-dispersible AMPS homopolymers, mention may be made, for example, of crosslinked or non-crosslinked polymers of sodium acrylamido-2-methylpropanesulfonate, such as that used in the commercial

product Simulgel 800 (CTFA name: Sodium Polyacryloyldimethyl Taurate), crosslinked ammonium acrylamido-2-methylpropanesulfonate polymers (INCI name: Ammonium polydimethyltauramide) such as those described in patent EP 0 815 928 B1 and such as the product sold under the trade name Hostacerin AMPS® by the company Clariant.

As water-soluble or water-dispersible AMPS copolymers, examples that may be mentioned include:

- crosslinked acrylamide/sodium acrylamido-2-methylpropanesulfonate copolymers, such as that used in the commercial product Sepigel 305 (CTFA name: Polyacrylamide/$C_{13}$-$C_{14}$ Isoparaffin/ Laureth-7) or that used in the commercial product sold under the name Simulgel 600 (CTFA name: Acrylamide/Sodium Acryloyldimethyltaurate/Isohexadecane/Polysorbate-80) by the company SEPPIC;
- copolymers of AMPS® and of vinylpyrrolidone or vinylformamide, such as that used in the commercial product sold under the name Aristoflex AVC® by the company Clariant (CTFA name: Ammonium acryloyldimethyltaurate/VP copolymer) but neutralized with sodium hydroxide or potassium hydroxide;
- copolymers of AMPS® and of sodium acrylate, for instance the AMPS/sodium acrylate copolymer, such as that used in the commercial product sold under the name Simulgel EG® by the company SEPPIC or under the trade name Sepinov EM (CTFA name: Hydroxyethyl acrylate/sodium acryloyldimethyltaurate copolymer);
- copolymers of AMPS® and of hydroxyethyl acrylate, for instance the AMPS®/hydroxyethyl acrylate copolymer, such as that used in the commercial product sold under the name Simulgel NS® by the company SEPPIC (CTFA name: Hydroxyethyl acrylate/sodium acryloyldimethyltaurate copolymer (and) squalane (and) polysorbate 60), or such as the product sold under the name Sodium acrylamido-2-methylpropanesulfonate/hydroxyethyl acrylate copolymer, such as the commercial product Sepinov EMT 10 (INCI name: Hydroxyethyl acrylate/sodium acryloyldimethyltaurate copolymer).

According to the invention, the aqueous gelling agent are copolymers of AMPS® and of hydroxyethyl acrylate, for instance the AMPS®/hydroxyethyl acrylate copolymer, such as that used in the commercial product sold under the name Simulgel NS® by the company SEPPIC (CTFA name: Hydroxyethyl acrylate/sodium acryloyldimethyltaurate copolymer (and) squalane (and) polysorbate 60), or such as the product sold under the name Sodium acrylamido-2-methylpropanesulfonate/hydroxyethyl acrylate copolymer, such as the commercial product Sepinov EMT 10 (INCI name: Hydroxyethyl acrylate/sodium acryloyldimethyltaurate copolymer).

Preferably, the product sold under the name Sodium acrylamido-2-methylpropanesulfonate/hydroxyethyl acrylate copolymer, such as the commercial product Sepinov EMT 10 (INCI name: Hydroxyethyl acrylate/sodium acryloyldimethyl taurate copolymer) is used as water-soluble or water-dispersible AMPS copolymers in accordance with the invention.

In general, an aqueous phase according to the invention may comprise from 0.1 % to 8 % by weight of solids, preferably 0.2 % to 5 % by weight and more preferentially from 0.7 % to 2.5 % by weight of polyacrylamide(s) and/or of crosslinked and/or neutralized 2-acrylamido-2-methylpropanesulfonic acid polymer(s) and copolymer(s) relative to its total weight.

## LIPOPHILIC GELLING AGENT

For the purposes of the present invention, the term *"lipophilic gelling agent"* means a compound that is capable of gelling the oily phase of the compositions according to the invention.

The gelling agent is lipophilic and is thus present in the oily phase of the composition.

The gelling agent is liposoluble or lipodispersible.

As emerges from the foregoing, the lipophilic gelling agent is advantageously chosen from hydrogen bonding polymers and more particularly polyamides, and mixtures thereof.

### Polyamides

The oily phase of a composition according to the invention comprises at least one polyamide chosen from hydrocarbon-based polyamides and silicone polyamides, and mixtures thereof.

This type of gelling agent is particularly advantageous with regard to the gloss and colour-revealing effect it affords when it is combined with a dyestuff.

It is therefore particularly advantageous for formulating cosmetic compositions intended for making up the skin and the lips.

Preferably, the total content of polyamide(s) is between 0.1% and 30% by weight expressed as solids, preferably between 0.1% and 20% by weight and preferably between 0.5% and 10% by weight relative to the total weight of the oily phase.

For the purposes of the invention, the term *"polyamide"* means a compound containing at least two repeating

amide units, preferably at least three repeating amide units and better still ten repeating amide units.

## A. Hydrocarbon-based polyamide

The term *"hydrocarbon-based polyamide"* means a polyamide formed essentially of, indeed even consisting of, carbon and hydrogen atoms, and optionally of oxygen or nitrogen atoms, and not comprising any silicon or fluorine atoms. It may contain alcohol, ester, ether, carboxylic acid, amine and/or amide groups.

For the purposes of the invention, the term *"functionalized chain"* means an alkyl chain comprising one or more functional groups or reagents chosen especially from hydroxyl, ether, ester, oxyalkylene and polyoxyalkylene groups.

Advantageously, this polyamide of the composition according to the invention has a weight-average molecular mass of less than 100 000 g/mol (especially ranging from 1000 to 100 000 g/mol), in particular less than 50 000 g/mol (especially ranging from 1000 to 50 000 g/mol) and more particularly ranging from 1000 to 30 000 g/mol, preferably from 2000 to 20 000 g/mol and better still from 2000 to 10 000 g/mol.

This polyamide is insoluble in water, in particular at 25°C.

According to a first embodiment of the invention, the polyamide used is a polyamide of formula (I):

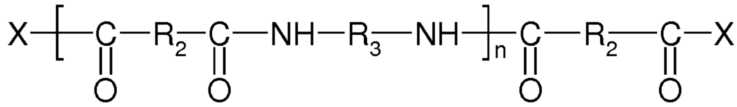

(I)

in which X represents a group $-N(R_1)_2$ or a group $-OR_1$ in which $R_1$ is a linear or branched $C_8$ to $C_{22}$ alkyl radical which may be identical or different, $R_2$ is a $C_{28}$-$C_{42}$ diacid dimer residue, $R_3$ is an ethylenediamine radical and n is between 2 and 5;

- and mixtures thereof.

According to a particular mode, the polyamide used is an amide-terminated polyamide of formula (Ia):

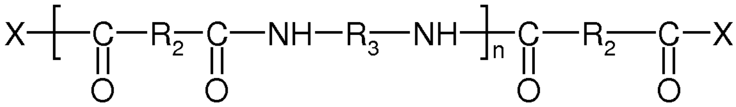

(Ia)

in which X represents a group $-N(R_1)_2$ in which $R_1$ is a linear or branched $C_8$ to $C_{22}$ alkyl radical which may be identical or different, $R_2$ is a $C_{28}$-$C_{42}$ diacid dimer residue, $R_3$ is an ethylenediamine radical and n is between 2 and 5;

- and mixtures thereof.

The oily phase of a composition according to the invention may also comprise, additionally in this case, at least one additional polyamide of formula (Ib):

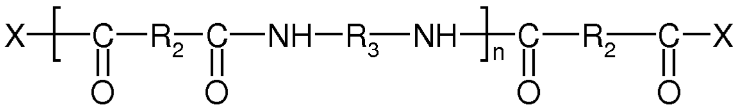

(Ib)

in which X represents a group $-OR_1$ in which $R_1$ is a linear or branched $C_8$ to $C_{22}$ and preferably $C_{16}$ to $C_{22}$ alkyl radical which may be identical or different, $R_2$ is a $C_{28}$-$C_{42}$ diacid dimer residue, $R_3$ is an ethylenediamine radical and n is between 2 and 5, mention may be made of the commercial products sold by the company Arizona Chemical under the names Uniclear 80 and Uniclear 100 or Uniclear 80 V, Uniclear 100 V and Uniclear 100 VG, the INCI name of which is Ethylenediamine/stearyl dimer dilinoleate copolymer.

**B. Silicone polyamide**

The silicone polyamides are preferably solid at room temperature (25°C) and atmospheric pressure (760 mmHg).

The silicone polyamides may preferentially be polymers comprising at least one unit of formula (III) or (IV):

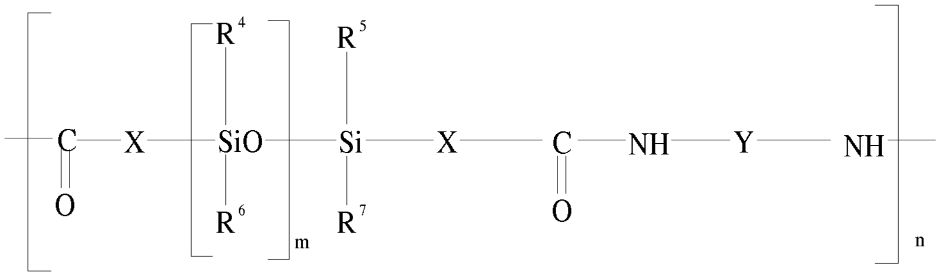

(III)

or

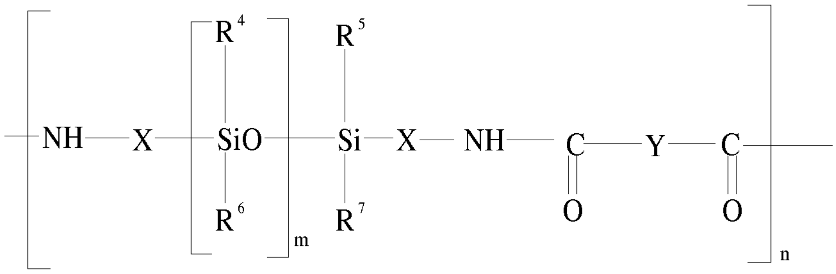

(IV)

in which:

- $R^4$, $R^5$, $R^6$ and $R^7$, which may be identical or different, represent a group chosen from:
  - saturated or unsaturated, $C_1$ to $C_{40}$ linear, branched or cyclic hydrocarbon-based groups, which may contain in their chain one or more oxygen, sulfur and/or nitrogen atoms, and which may be partially or totally substituted with fluorine atoms,
  - $C_6$ to $C_{10}$ aryl groups, optionally substituted with one or more $C_1$ to $C_4$ alkyl groups,
  - polyorganosiloxane chains possibly containing one or more oxygen, sulfur and/or nitrogen atoms,
- the groups X, which may be identical or different, represent a linear or branched $C_1$ to $C_{30}$ alkylenediyl group, possibly containing in its chain one or more oxygen and/or nitrogen atoms,
- Y is a saturated or unsaturated $C_1$ to $C_{50}$ linear or branched alkylene, arylene, cycloalkylene, alkylarylene or arylalkylene divalent group, which may comprise one or more oxygen, sulfur and/or nitrogen atoms, and/or may bear as substituent one of the following atoms or groups of atoms: fluorine, hydroxyl, $C_3$ to $C_8$ cycloalkyl, $C_1$ to $C_{40}$ alkyl, $C_5$ to $C_{10}$ aryl, phenyl optionally substituted with one to three $C_1$ to $C_3$ alkyl, $C_1$ to $C_3$ hydroxyalkyl and $C_1$ to $C_6$ aminoalkyl groups, or Y represents a group corresponding to the formula:

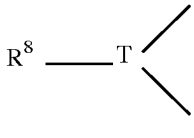

in which:

  - T represents a linear or branched, saturated or unsaturated, $C_3$ to $C_{24}$ trivalent or tetravalent hydrocarbon-based group optionally substituted with a polyorganosiloxane chain, and possibly containing one or more atoms

chosen from O, N and S, or T represents a trivalent atom chosen from N, P and Al, and

- R8 represents a linear or branched $C_1$-$C_{50}$ alkyl group or a polyorganosiloxane chain, possibly comprising one or more ester, amide, urethane, thiocarbamate, urea, thiourea and/or sulfonamide groups, which may possibly be linked to another chain of the polymer,

• n is an integer ranging from 2 to 500 and preferably from 2 to 200, and m is an integer ranging from 1 to 1000, preferably from 1 to 700 and even better still from 6 to 200.

According to a particular mode, the silicone polyamide comprises at least one unit of formula (III) in which m ranges from 50 to 200, in particular from 75 to 150 and is preferably about 100.

More preferably, $R^4$, $R^5$, $R^6$ and $R^7$ independently represent a linear or branched $C_1$ to $C_{40}$ alkyl group, preferably a group $CH_3$, $C_2H_5$, n-$C_3H_7$ or isopropyl in formula (III).

As examples of silicone polymers that may be used, mention may be made of one of the silicone polyamides obtained in accordance with Examples 1 to 3 of document US-A-5 981 680.

Mention may be made of the compounds sold by the company Dow Corning under the name DC 2-8179 (DP 100) and DC 2-8178 (DP 15), the INCI name of which is Nylon-611/dimethicone copolymers, i.e. Nylon-611/dimethicone copolymers. The silicone polymers and/or copolymers advantageously have a temperature of transition from the solid state to the liquid state ranging from 45°C to 190°C. Preferably, they have a temperature of transition from the solid state to the liquid state ranging from 70 to 130°C and better still from 80°C to 105°C.

Preferably, the total content of polyamide(s) and/or silicone polyamide(s) is between 0.5% and 25% by weight of solids, in particular from 2% to 20% by weight and preferably between 2% and 12% by weight relative to the total weight of the oily phase.

Advantageously, the hydrogen bonding polymer is chosen from ethylenediamine/stearyl dimer dilinoleate copolymer and Nylon-611/dimethicone copolymers.

## HYDROPHILIC GELLING AGENT(S)/LIPOPHILIC GELLING AGENT(S) SYSTEMS

As non-limiting illustrations of hydrophilic gelling agent(s)/lipophilic gelling agent(s) systems that are most particularly suitable for use in the invention, mention may be made of the system of synthetic polymeric hydrophilic gelling agent(s)/ hydrogen bonding polymer(s).

As preferred hydrophilic gelling agents of synthetic polymeric type that are suitable for use in the invention, mention may be made more particularly of AMPS and 2-acrylamido-2-methylpropanesulfonic acid copolymers and in particular copolymers of AMPS® and of hydroxyethyl acrylate, for instance the AMPS®/hydroxyethyl acrylate copolymer, such as that used in the commercial product sold under the name Simulgel NS® by the company SEPPIC (CTFA name: Hydroxyethyl acrylate/sodium acryloyldimethyltaurate copolymer (and) squalane (and) polysorbate 60), or such as the product sold under the name Sodium acrylamido-2-methylpropanesulfonate/hydroxyethyl acrylate copolymer, such as the commercial product Sepinov EMT 10 (INCI name: Hydroxyethyl acrylate/sodium acryloyldimethyltaurate copolymer).

As preferred lipophilic hydrogen bonding polymers that are suitable for use in the invention, mention may be made more particularly of hydrocarbon-based polyamides, for instance the commercial products sold by the company Arizona Chemical under the names Uniclear 80 and Uniclear 100 or Uniclear 80 V, Uniclear 100 V and Uniclear 100 VG, the INCI name of which is ethylenediamine/stearyl dimer dilinoleate copolymer, and silicone polyamides, for instance the compounds sold by the company Dow Corning under the name DC 2-8179 (DP 100) and DC 2-8178 (DP 15), the INCI name of which is Nylon-611/dimethicone copolymers, i.e. Nylon-611/dimethicone copolymers.

Thus, a composition according to the invention may advantageously comprise as hydrophilic gelling agent(s)/lipophilic gelling agent(s) systems, a system of the type:

- copolymer of 2-acrylamido-2-methylpropanesulfonic acid and of hydroxyethyl acrylate/hydrocarbon-based polyamide; or
- copolymer of 2-acrylamido-2-methylpropanesulfonic acid and of hydroxyethyl acrylate/silicone polyamide.

In particular, it is a system of the type:

- copolymer of 2-acrylamido-2-methylpropanesulfonic acid and of hydroxyethyl acrylate/ethylenediamine/stearyl dimer dilinoleate copolymer; and
- copolymer of 2-acrylamido-2-methylpropanesulfonic acid and of hydroxyethyl acrylate/Nylon-611/dimethicone copolymer.

## AQUEOUS PHASE

The aqueous phase of a composition according to the invention comprises water and optionally a water-soluble solvent.

In the present invention, the term *"water-soluble solvent"* denotes a compound that is liquid at room temperature and water-miscible (miscibility with water of greater than 50% by weight at 25°C and atmospheric pressure).

The water-soluble solvents that may be used in the composition of the invention may also be volatile.

Among the water-soluble solvents that may be used in the composition in accordance with the invention, mention may be made especially of lower monoalcohols containing from 1 to 5 carbon atoms, such as ethanol and isopropanol, glycols containing from 2 to 8 carbon atoms, such as ethylene glycol, propylene glycol, 1,3-butylene glycol and dipropylene glycol, $C_3$ and $C_4$ ketones and $C_2$-$C_4$ aldehydes.

The aqueous phase (water and optionally the water-miscible solvent) may be present in the composition in a content ranging from 5% to 95%, better still from 30% to 80% by weight and preferably from 40% to 75% by weight relative to the total weight of the said composition.

According to another embodiment variant, the aqueous phase of a composition according to the invention may comprise at least one $C_2$-$C_{32}$ polyol.

For the purposes of the present invention, the term *"polyol"* should be understood as meaning any organic molecule comprising at least two free hydroxyl groups.

Preferably, a polyol in accordance with the present invention is present in liquid form at room temperature.

A polyol that is suitable for use in the invention may be a compound of linear, branched or cyclic, saturated or unsaturated alkyl type, bearing on the alkyl chain at least two -OH functions, in particular at least three -OH functions and more particularly at least four -OH functions.

The polyols advantageously suitable for the formulation of a composition according to the present invention are those exhibiting in particular from 2 to 32 carbon atoms and preferably from 3 to 16 carbon atoms.

Advantageously, the polyol may be chosen, for example, from ethylene glycol, pentaerythritol, trimethylolpropane, propylene glycol, 1,3-propanediol, butylene glycol, isoprene glycol, pentylene glycol, hexylene glycol, glycerol, polyglycerols such as glycerol oligomers, for instance diglycerol, and polyethylene glycols, and mixtures thereof.

According to a preferred embodiment of the invention, the said polyol is chosen from ethylene glycol, pentaerythritol, trimethylolpropane, propylene glycol, glycerol, polyglycerols and polyethylene glycols, and mixtures thereof.

According to a particular embodiment, the composition of the invention may comprise at least propylene glycol.

According to another particular embodiment, the composition of the invention may comprise at least glycerol.

## OILY PHASE

For the purposes of the invention, an oily phase comprises at least one oil.

The term *"oil"* means any fatty substance that is in liquid form at room temperature and atmospheric pressure.

An oily phase that is suitable for preparing the cosmetic compositions according to the invention may comprise hydrocarbon-based oils, silicone oils, fluoro oils or non-fluoro oils, or mixtures thereof.

The oils may be volatile or non-volatile.

They can be of animal, vegetable, mineral or synthetic origin. According to one embodiment variant, oils of plant origin are preferred.

For the purposes of the present invention, the term *"non-volatile oil"* means an oil with a vapour pressure of less than 0.13 Pa.

For the purposes of the present invention, the term *"silicone oil"* is intended to mean an oil comprising at least one silicon atom, and in particular at least one Si-O group.

The term "*fluoro oil*" means an oil comprising at least one fluorine atom.

The term "*hydrocarbon-based oil*" means an oil mainly containing hydrogen and carbon atoms.

The oils may optionally comprise oxygen, nitrogen, sulfur and/or phosphorus atoms, for example in the form of hydroxyl or acid radicals.

For the purposes of the invention, the term *"volatile oil"* means any oil that is capable of evaporating on contact with the skin in less than one hour, at room temperature and atmospheric pressure. The volatile oil is a volatile cosmetic compound, which is liquid at room temperature, especially having a nonzero vapour pressure, at room temperature and atmospheric pressure, in particular having a vapour pressure ranging from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1,300 Pa (0.01 to 10 mmHg).

## Volatile oils

The volatile oils may be hydrocarbon-based oils or silicone oils.

Among the volatile hydrocarbon-based oils containing from 8 to 16 carbon atoms, mention may be made especially of branched $C_8$-$C_{16}$ alkanes, for instance $C_8$-$C_{16}$ isoalkanes (also known as isoparaffins), isododecane, isodecane, isohexadecane and, for example, the oils sold under the trade names Isopar or Permethyl, branched $C_8$-$C_{16}$ esters, for instance isohexyl neopentanoate, and mixtures thereof. Preferably, the volatile hydrocarbon-based oil is chosen from volatile hydrocarbon-based oils containing from 8 to 16 carbon atoms, and mixtures thereof, in particular from isododecane, isodecane and isohexadecane, and is especially isohexadecane.

Mention may also be made of volatile linear alkanes comprising from 8 to 16 carbon atoms, in particular from 10 to 15 carbon atoms and more particularly from 11 to 13 carbon atoms, for instance n-dodecane (C12) and n-tetradecane (C14) sold by Sasol under the respective references Parafol 12-97 and Parafol 14-97, and also mixtures thereof, the undecane-tridecane mixture, mixtures of n-undecane (C11) and of n-tridecane (C13) obtained in Examples 1 and 2 of patent application WO 2008/155 059 from the company Cognis, and mixtures thereof.

Volatile silicone oils that may be mentioned include linear volatile silicone oils such as hexamethyldisiloxane, octamethyltrisiloxane, decamethyltetrasiloxane, tetradecamethylhexasiloxane, hexadecamethylheptasiloxane and dodecamethylpentasiloxane.

Volatile cyclic silicone oils that may be mentioned include hexamethylcyclotrisiloxane, octamethylcyclotetrasiloxane, decamethylcyclopentasiloxane and dodecamethylcyclohexasiloxane.

## Non-volatile oils

The non-volatile oils may be chosen especially from non-volatile hydrocarbon-based, fluoro and/or silicone oils.

Non-volatile hydrocarbon-based oils that may especially be mentioned include:

- hydrocarbon-based oils of animal origin,
- hydrocarbon-based oils of plant origin, synthetic ethers containing from 10 to 40 carbon atoms, such as dicaprylyl ether,
- synthetic esters, for instance the oils of formula $R_1COOR_2$, in which $R_1$ represents a linear or branched fatty acid residue containing from 1 to 40 carbon atoms and $R_2$ represents a hydrocarbon-based chain, which is especially branched, containing from 1 to 40 carbon atoms, on condition that $R_1 + R_2 \geq 10$. The esters may be chosen especially from fatty alcohol and fatty acid esters, for instance cetostearyl octanoate, isopropyl alcohol esters such as isopropyl myristate or isopropyl palmitate, ethyl palmitate, 2-ethylhexyl palmitate, isopropyl stearate, octyl stearate, hydroxylated esters, for instance isostearyl lactate or octyl hydroxystearate, alkyl or polyalkyl ricinoleates, hexyl laurate, neopentanoic acid esters, for instance isodecyl neopentanoate or isotridecyl neopentanoate, and isononanoic acid esters, for instance isononyl isononanoate or isotridecyl isononanoate,

- polyol esters and pentaerythritol esters, for instance dipentaerythrityl tetrahydroxystearate/tetraiso stearate,
- fatty alcohols that are liquid at room temperature, with a branched and/or unsaturated carbon-based chain containing from 12 to 26 carbon atoms, for instance 2-octyldodecanol, isostearyl alcohol and oleyl alcohol,
- $C_{12}$-$C_{22}$ higher fatty acids, such as oleic acid, linoleic acid, linolenic acid, and mixtures thereof,
- non-phenyl silicone oils, for instance caprylyl methicone, and
- phenyl silicone oils, for instance phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates, dimethicones or phenyl trimethicone with a viscosity of less than or equal to 100 cSt, and trimethyl pentaphenyl trisiloxane, and mixtures thereof; and also mixtures of these various oils.

Preferably, a composition according to the invention comprises volatile and/or non volatile silicone oils. Such silicone oils are particularly appreciated when the oily gelling agent is an organopolysiloxane polymer.

A composition according to the invention may comprise from 5% to 95% by weight, better still from 5% to 40% by weight and preferably from 7% to 35% by weight of oil(s) relative to the total weight of the said composition.

As mentioned above, the gelled oily phase according to the invention may have a threshold stress of greater than 1.5 Pa and preferably greater than 10 Pa. This threshold stress value reflects a gel-type texture of this oily phase.

## DYESTUFFS

A composition according to the invention may also comprise at least one particulate or non-particulate, water-soluble or water-insoluble dyestuff, preferably in a proportion of at least 0.01% by weight relative to the total weight of

the composition.

For obvious reasons, this amount is liable to vary significantly with regard to the intensity of the desired colour effect and of the colour intensity afforded by the dyestuffs under consideration, and its adjustment clearly falls within the competence of a person skilled in the art.

A composition according to the invention may comprise from 0.01% to 15% by weight, especially from 0.1% to 15% by weight, in particular from 1% to 15% by weight and preferably from 5% to 15% by weight of dyestuffs relative to the total weight of the said composition. As stated above, the dyestuffs that are suitable for use in the invention may be water-soluble, but may also be liposoluble.

Advantageously, a composition according to the invention may comprise from 0.01% to 25% by weight, especially from 0.1% to 25% by weight, in particular from 1% to 20% by weight and preferably from 5% to 15% by weight of dyestuffs relative to the total weight of the said composition.

For the purposes of the invention, the term "*water-soluble dyestuff*" means any natural or synthetic, generally organic compound, which is soluble in an aqueous phase or in water-miscible solvents, and which is capable of imparting colour.

As water-soluble dyes that are suitable for use in the invention, mention may be made especially of synthetic or natural water-soluble dyes, for instance FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanin (beetroot), carmine, copper chlorophylline, methylene blue, anthocyanins (enocianin, black carrot, hibiscus and elder), caramel and riboflavin.

The water-soluble dyes are, for example, beetroot juice and caramel.

For the purposes of the invention, the term "*liposoluble dyestuff*" means any natural or synthetic, generally organic compound, which is soluble in an oily phase or in solvents that are miscible with a fatty substance, and which is capable of imparting colour.

As liposoluble dyes that are suitable for use in the invention, mention may be made especially of synthetic or natural liposoluble dyes, for instance DC Red 17, DC Red 21, DC Red 27, DC Green 6, DC Yellow 11, DC Violet 2, DC Orange 5, Sudan red, carotenes (β-carotene, lycopene), xanthophylls (capsanthin, capsorubin, lutein), palm oil, Sudan brown, quinoline yellow, annatto and curcumin.

The particulate dyestuffs may be present in a proportion of from 0.01% to 15% by weight relative to the total weight of the composition containing them.

They may especially be pigments, nacres and/or particles with metallic tints.

The term *"pigments"* should be understood as meaning white or coloured, mineral or organic particles that are insoluble in an aqueous solution, which are intended to colour and/or opacify the composition containing them.

Advantageously, a composition according to the invention may comprise from 0.01% to 25% by weight, especially from 0.1% to 25% by weight, in particular from 1% to 20% by weight and preferably from 5% to 15% by weight of pigments relative to the total weight of the said composition.

Preferably, when a composition according to the invention is a make-up composition, it may comprise at least 5%, and more preferably at least 10% by weight of pigments relative to the total weight of the said composition.

The pigments may be white or coloured, and mineral and/or organic.

As mineral pigments that may be used in the invention, mention may be made of titanium oxide, titanium dioxide, zirconium oxide, zirconium dioxide, cerium oxide or cerium dioxide and also zinc oxide, iron oxide or chromium oxide, ferric blue, manganese violet, ultramarine blue and chromium hydrate, and mixtures thereof.

It may also be a pigment having a structure that may be, for example, of sericite/brown iron oxide/titanium dioxide/silica type. Such a pigment is sold, for example, under the reference Coverleaf NS or JS by the company Chemicals and Catalysts, and has a contrast ratio in the region of 30.

They may also be pigments having a structure that may be, for example, of silica microsphere type containing iron oxide. An example of a pigment having this structure is the product sold by the company Miyoshi under the reference PC Ball PC-LL-100 P, this pigment being constituted of silica microspheres containing yellow iron oxide.

Advantageously, the pigments in accordance with the invention are iron oxides and/or titanium dioxides.

The term *"nacres"* should be understood as meaning iridescent or non-iridescent coloured particles of any shape, especially produced by certain molluscs in their shell or alternatively synthesized, which have a colour effect via optical interference.

A composition according to the invention may comprise from 0% to 15% by weight of nacres relative to the total weight of the said composition.

The nacres may be chosen from nacreous pigments such as titanium mica coated with an iron oxide, titanium mica coated with bismuth oxychloride, titanium mica coated with chromium oxide, titanium mica coated with an organic dye and also nacreous pigments based on bismuth oxychloride. They may also be mica particles at the surface of which are superposed at least two successive layers of metal oxides and/or of organic dyestuffs.

Examples of nacres that may also be mentioned include natural mica coated with titanium oxide, with iron oxide, with natural pigment or with bismuth oxychloride.

Among the nacres available on the market, mention may be made of the nacres Timica, Flamenco and Duochrome (based on mica) sold by the company Engelhard, the Timiron nacres sold by the company Merck, the Prestige mica-based nacres sold by the company Eckart, and the Sunshine synthetic mica-based nacres sold by the company Sun Chemical.
The nacres may more particularly have a yellow, pink, red, bronze, orangey, brown, gold and/or coppery colour or tint.
Advantageously, the nacres in accordance with the invention are micas coated with titanium dioxide or with iron oxide, and also bismuth oxychloride.
For the purposes of the present invention, the term *"particles with a metallic tint"* means any compound whose nature, size, structure and surface finish allow it to reflect the incident light, especially in a non-iridescent manner.
The particles with a metallic tint that may be used in the invention are in particular chosen from:

- particles of at least one metal and/or of at least one metal derivative;
- particles comprising a single-material or multi-material organic or mineral substrate, at least partially coated with at least one layer with a metallic tint comprising at least one metal and/or at least one metal derivative; and
- mixtures of the said particles.

Among the metals that may be present in the said particles, mention may be made, for example, of Ag, Au, Cu, Al, Ni, Sn, Mg, Cr, Mo, Ti, Zr, Pt, Va, Rb, W, Zn, Ge, Te and Se, and mixtures or alloys thereof. Ag, Au, Cu, Al, Zn, Ni, Mo and Cr, and mixtures or alloys thereof (for example bronzes and brasses) are preferred metals.
The term *"metal derivatives"* denotes compounds derived from metals, especially oxides, fluorides, chlorides and sulfides.
Illustrations of these particles that may be mentioned include aluminum particles, such as those sold under the names Starbrite 1200 EAC® by the company Siberline and Metalure® by the company Eckart and glass particles coated with a metallic layer, especially those described in documents JP-A-09188830, JP-A-10158450, JP-A-10158541, JP-A-07258460 and JP-A-05017710.

Hydrophobic treatment of the dyestuffs

The pulverulent dyestuffs as described previously may be totally or partially surface-treated, with a hydrophobic agent, to make them more compatible with the oily phase of the composition of the invention, especially so that they have good wettability with oils. Thus, these treated pigments are well dispersed in the oily phase.
Hydrophobic-treated pigments are described especially in document EP-A-1 086 683.
The hydrophobic-treatment agent may be chosen from silicones such as methicones, dimethicones and perfluoroalkylsilanes; fatty acids, for instance stearic acid; metal soaps, for instance aluminium dimyristate, the aluminium salt of hydrogenated tallow glutamate; perfluoroalkyl phosphates; polyhexafluoropropylene oxides; perfluoropolyethers; amino acids; N-acylamino acids or salts thereof; lecithin, isopropyl triisostearyl titanate, isostearyl sebacate, and mixtures thereof.
The term *"alkyl"* mentioned in the compounds cited above especially denotes an alkyl group containing from 1 to 30 carbon atoms and preferably containing from 5 to 16 carbon atoms.

**FILLERS**

For the purposes of the present invention, the term *"fillers"* should be understood as meaning colourless or white solid particles of any form, which are in an insoluble and dispersed form in the medium of the composition.
These fillers, of mineral or organic, natural or synthetic nature, give the composition containing them softness and give the makeup result a matt effect and uniformity.
Preferably, a composition of the invention comprises fillers, particularly when it is dedicated to provide a high coverage.
In particular, a composition according to the invention may comprise from 2% to 35% by weight, especially from 5% to 35% by weight, in particular from 5% to 20% by weight.
According to one embodiment of the invention, a composition may comprise solid particles such as pigments and/or fillers.
Advantageously, a composition according to the invention may comprise from 0.01% to 25% by weight, especially from 0.1% to 25% by weight, in particular from 1% to 20% by weight and preferably from 5% to 15% by weight of solid particles relative to the total weight of the said composition.
Preferably, when a composition according to the invention is a make-up composition, it may comprise at least 5%, and more preferably at least 10% by weight of solid particles relative to the total weight of the said composition.

## DISPERSANT

Advantageously, a composition according to the invention may also comprise a dispersant.
Such a dispersant may be a surfactant, an oligomer, a polymer or a mixture of several thereof.
According to one particular embodiment, a dispersant in accordance with the invention is a surfactant.

## ACTIVE AGENT

For a particular care application, a composition according to the invention may comprise at least one moisturizer (also known as a humectant).
Preferably, such moisturizer is glycerol.
The moisturizer(s) could be present in the composition in a content ranging from 0.1% to 15% by weight, especially from 0.5% to 10% by weight or even from 1% to 6% by weight, relative to the total weight of the said composition.
As other active agents that may be used in the composition of the invention, examples that may be mentioned include vitamins and sunscreens, and mixtures thereof.
Preferably, a composition of the invention comprises at least one active agent.
It is a matter of routine for those skilled in the art to adjust the nature and amount of the additives present in the compositions in accordance with the invention such that the desired cosmetic properties thereof are not thereby affected.
According to one embodiment, a composition of the invention may advantageously be in the form of a foundation.
According to one embodiment, a composition of the invention may advantageously be in the form of a composition for making up the skin and especially the face. It may thus be an eyeshadow or a face powder.
According to another embodiment, a composition of the invention may advantageously be in the form of a lip product, and in particular a lipstick.
According to another embodiment, a composition of the invention may advantageously be in the form of a composition for caring for the skin of the body or the face, in particular the face.
According to another embodiment, a composition of the invention may be in the form of a product for the eyelashes, in particular a mascara.
Such compositions are especially prepared according to the general knowledge of a person skilled in the art.
Throughout the description, including the claims, the term "*comprising a*" should be understood as being synonymous with *"comprising at least one",* unless otherwise specified.
The expressions *"between ... and* ..." and *"ranging from ... to* ..." should be understood as meaning limits included, unless otherwise specified.
The invention is illustrated in greater detail by the examples and figures presented below. Unless otherwise mentioned, the amounts indicated are expressed as weight percentages.

## Methodology for the oscillating dynamic rheology measurements

These are rheological measurements in the harmonic regime, which measure the elastic modulus.
The measurements are taken using a Haake RS600 rheometer on a product at rest, at 25°C with a plate-plate rotor Ø 60 mm and a 2 mm gap.
The measurements in the harmonic regime make it possible to characterize the viscoelastic properties of the products. The technique consists in subjecting a material to a stress that varies sinusoidally over time and in measuring the response of the material to this stress. In a region in which the behaviour is linearly viscoelastic (zone in which the strain is proportional to the stress), the stress ($\tau$) and the strain ($\gamma$) are two sinusoidal functions of time that are written in the following manner:

$$\tau(t) = \tau_0 \sin(\omega t)$$

$$\gamma(t) = \gamma_0 \sin(\omega t + \delta)$$

in which:

$\tau_0$ represents the maximum amplitude of the stress (Pa);
$\gamma_0$ represents the maximum amplitude of the strain (-);
$\omega = 2\Pi N$ represents the angular frequency (rad.s$^{-1}$) with N representing the frequency (Hz); and

$\delta$ represents the phase angle of the stress relative to the strain (rad).

Thus, the two functions have the same angular frequency, but they are dephased by an angle $\delta$. According to the phase angle $\delta$ between $\tau(t)$ and $\gamma(t)$, the behaviour of the system may be assessed:

- if $\delta = 0$, the material is purely elastic;
- if $\delta = \Pi/2$, the material is purely viscous (Newtonian fluid); and
- if $0 < \delta < \Pi/2$, the material is viscoelastic.

In general, the stress and the strain are written in complex form:

$$\tau^*(t) = \tau_0\, e^{i\omega t}$$

$$\gamma^*(t) = \gamma_0\, e^{(i\omega t + \delta)}$$

A complex stiffness modulus, representing the overall resistance of the material to the strain, whether it is of elastic or viscous origin, is then defined by:

$$G^* = \tau^* / \gamma^* = G' + iG''$$

in which:

G' is the storage modulus or elastic modulus, which characterizes the energy stored and totally restituted in the course of a cycle, $G' = (\tau_0/\gamma_0) \cos \delta$; and
G" is the loss modulus or viscous modulus, which characterizes the energy dissipated by internal friction in the course of a cycle, $G'' = (\tau_0/\gamma_0) \sin \delta$.

The parameter retained is the mean stiffness modulus G* recorded at the plateau measured at a frequency of 1 Hz.

**Example 1**

Foundation formulations in accordance with the invention are prepared from the phases described below.

1) Preparation of the aqueous phase A1

The aqueous phase is prepared from the compounds that follow in the weight proportions stated in the table below.

*Phase A1:*

| Compounds | Weight % Phase A1 |
|---|---|
| Water | qs 100 |
| Glycerol | 10.00 |
| Butylene glycol | 6.25 |
| Phenoxyethanol | 0.63 |
| Caprylyl glycol | 0.63 |
| Hydroxyethyl acrylate/sodium acryloyldimethyltaurate copolymer (Sepinov® EMT 10 sold by the company SEPPIC) | 1.74 |

The water, glycerol, butylene glycol, phenoxyethanol and caprylyl glycol are weighed out in a beaker and stirred

using a Rayneri blender at room temperature.

The hydroxyethyl acrylate/sodium acryloyldimethyltaurate copolymer is added with stirring at room temperature. The stirring is adjusted so as not to incorporate air into the mixture.

The mixture is stirred moderately for about 10 minutes at room temperature.

2) Preparation of the oily phases

The oily phases are prepared from the compounds that follow in the weight proportions stated in the tables below.

*Phase B1:*

| **Compounds** | **Weight % Phase B1** |
|---|---|
| Yellow iron oxide | 4.92 |
| Red iron oxide | 1.00 |
| Black iron oxide | 0.35 |
| Titanium dioxide | 31.24 |
| Isononyl isononanoate | qs 100 |
| Ethylenediamine/stearyl dimer dilinoleate copolymer (Uniclear® 100 VG sold by the company Arizona Chemical) | 12 |

The pigments are ground with 15% of the oils using a three-roll mill.

The ground material, the remainder of the oils and the ethylenediamine/stearyl dimer dilinoleate copolymer are placed in a beaker and stirred using a Rayneri blender.

The mixture is heated to 100°C until dissolution of the ethylenediamine/stearyl dimer dilinoleate copolymer is complete (about 20 to 25 minutes).

The mixture is allowed to cool to room temperature.

*Phase B2:*

| **Compounds** | **Weight % Phase B2** |
|---|---|
| Yellow iron oxide | 4.92 |
| Red iron oxide | 1.00 |
| Black iron oxide | 0.35 |
| Titanium dioxide | 31.24 |
| Isononyl isononanoate | qs 100 |
| Nylon-611/dimethicone copolymer (Dow Corning 2-8179 Gellant sold by the company Dow Corning) | 11.00 |

The pigments are ground with 15% of the oils using a three-roll mill.

The ground material, the remainder of the oils and the Nylon-611/dimethicone copolymer are placed in a beaker and stirred using a Rayneri blender.

The mixture is heated to 100°C until dissolution of the Nylon-611/dimethicone copolymer is complete (about 20 to 25 minutes).

The mixture is allowed to cool to room temperature.

3) Preparation of foundation formulations

These formulations are obtained by mixing several phases intended to form the foundations in accordance with the invention, in the proportions described below in Table 1.

The aqueous and oily phases are weighed out and then stirred moderately for about 10 minutes.

The combination of the various phases is established as a function of the desired properties.

*Table 1*

| Formulations | Technical performance quality (ies) obtained | Weight % Phase A1 | Weight % Phase B1 | Weight % Phase B2 |
|---|---|---|---|---|
| Formulatio n 1 | Freshness and radiance/luminous | 60.00 | 40.00 | |
| Formulatio n 2 | Freshness and radiance/luminous | 60.00 | | 40.00 |

Formulation 1 is thick and has a foam appearance. The formulation spreads well, and the deposit gives average coverage and is homogeneous. The skin finish is satiny and slightly tacky.

Formulation 2 is creamy and has a foam appearance. The formulation spreads very well and gives a satiny/glossy deposit, which is homogeneous and slightly tacky.

## Claims

1. Cosmetic composition for making up and/or caring for keratin materials, in particular the skin and/or the lips, comprising:

   - at least one aqueous phase gelled with at least one hydrophilic gelling agent chosen from synthetic polymeric gelling agents; and
   - at least one oily phase gelled with at least one hydrogen bonding polymer chosen from hydrocarbon-based polyamides and silicone polyamides, and mixtures thereof;

   the said phases forming therein a macroscopically homogeneous mixture,
   wherein the composition contains, as hydrophilic gelling agent(s)/lipophilic gelling agent(s) system, a system chosen from:

   - copolymer of 2-acrylamido-2-methylpropanesulfonic acid and of hydroxyethyl acrylate/hydrocarbon-based polyamide; and
   - copolymer of 2-acrylamido-2-methylpropanesulfonic acid and of hydroxyethyl acrylate/silicone polyamide.

2. Composition according to the preceding claim, containing at least one dyestuff, preferably present at least in the gelled oily phase.

3. Composition according to any one of the preceding claims, containing, as hydrophilic gelling agent(s)/lipophilic gelling agent(s) system, a system chosen from:

   - copolymer of 2-acrylamido-2-methylpropanesulfonic acid and of hydroxyethyl acrylate/ethylenediamine/stearyl dimer dilinoleate copolymer; and
   - copolymer of 2-acrylamido-2-methylpropanesulfonic acid and of hydroxyethyl acrylate/Nylon-611/dimethicone copolymer.

4. Composition according to any one of the preceding claims, containing the aqueous and oily phases in an aqueous phase/oily phase weight ratio of from 95/5 to 5/95, preferably from 30/70 to 80/20, especially ranging from 60/40 to 80/20, preferably ranging from 60/40 to 70/30 and more preferably 60/40 or 70/30.

5. Composition according to any one of the preceding claims, which is in the form of a foundation, a face powder, an eyeshadow, a lipstick, a mascara and/or a care composition.

6. Composition according to any one of the preceding claims, further comprising solid particles such as pigments and/or fillers and in particular comprising from 0.01% to 25% by weight, especially from 0.1% to 25% by weight, in particular from 1% to 20% by weight and preferably from 5% to 15% by weight of solid particles relative to the total weight of the said composition.

7. Composition according to any one of the preceding claims, further comprising volatile and/or non volatile silicone oils.

**8.** Composition according to any one of the preceding claims, further comprising a moisturizer, and preferably glycerol.

**9.** Process for preparing a cosmetic composition for making up and/or caring for keratin materials, in particular the skin and/or the lips, comprising at least one step of mixing:

- at least one aqueous phase gelled with at least one hydrophilic gelling agent; and
- at least one oily phase gelled with at least one hydrogen bonding polymer;

under conditions suitable for obtaining a macroscopically homogeneous mixture the hydrophilic gelling agent(s)/lipophilic gelling agent(s) system being chosen from:

- copolymer of 2-acrylamido-2-methylpropanesulfonic acid and of hydroxyethyl acrylate/hydrocarbon-based polyamide; and
- copolymer of 2-acrylamido-2-methylpropanesulfonic acid and of hydroxyethyl acrylate/silicone polyamide.

**10.** Process according to the preceding claim, comprising a step of mixing at least three or even more gelled phases.

**11.** Process according to either of Claims 9 and 10, in which the mixing is performed at room temperature.

**12.** Cosmetic kit for making up and/or caring for keratin materials, in particular the skin and/or the lips, comprising, in separate containers, at least one aqueous phase gelled with at least one hydrophilic gelling agent, and at least one oily phase gelled with at least one hydrogen bonding polymer, and also instructions for using the extemporaneous mixtures, the hydrophilic gelling agent(s)/lipophilic gelling agent(s) system being chosen from:

- copolymer of 2-acrylamido-2-methylpropanesulfonic acid and of hydroxyethyl acrylate/hydrocarbon-based polyamide; and
- copolymer of 2-acrylamido-2-methylpropanesulfonic acid and of hydroxyethyl acrylate/silicone polyamide.

**13.** Device for making up and/or caring for keratin materials, in particular the skin and/or the lips, comprising at least:

- two separate containers containing, respectively, an aqueous phase gelled with at least one hydrophilic gelling agent, and an oily phase gelled with at least one hydrogen bonding polymer, the hydrophilic gelling agent(s)/lipophilic gelling agent(s) system being chosen from:
- copolymer of 2-acrylamido-2-methylpropanesulfonic acid and of hydroxyethyl acrylate/hydrocarbon-based polyamide; and
- copolymer of 2-acrylamido-2-methylpropanesulfonic acid and of hydroxyethyl acrylate/silicone polyamide;
- a distinct chamber for mixing the said containers, comprising an aperture configured to allow the introduction of the said phases to be mixed; and
- a means for distributing a macroscopically homogeneous mixture of the two phases.

**14.** Cosmetic process for making up and/or caring for keratin materials, in particular the skin and/or the lips, comprising at least one step which consists in applying to the said keratin material a composition as defined according to any one of Claims 1 to 8.

**15.** Cosmetic process for making up and/or caring for a keratin material, in particular the skin and/or the lips, comprising at least the application to the said material of a composition obtained by extemporaneous mixing, before application or at the time of application to the said keratin material, of at least one aqueous phase gelled with at least one hydrophilic gelling agent, and at least one oily phase gelled with at least one hydrogen bonding polymer, said composition containing, as hydrophilic gelling agent(s)/lipophilic gelling agent(s) system, a system chosen from:

- copolymer of 2-acrylamido-2-methylpropanesulfonic acid and of hydroxyethyl acrylate/hydrocarbon-based polyamide; and
- copolymer of 2-acrylamido-2-methylpropanesulfonic acid and of hydroxyethyl acrylate/silicone polyamide.

**Patentansprüche**

**1.** Kosmetische Zusammensetzung zum Schminken und/oder Pflegen von Keratinmaterialien, insbesondere der Haut

und/oder der Lippen, Folgendes umfassend:

- mindestens eine wässrige Phase, die mit mindestens einem hydrophilen Geliermittel, ausgewählt aus synthetischen polymeren Geliermitteln, geliert ist; und
- mindestens eine Ölphase, die mit mindestens einem wasserstoffbindenden Polymer, ausgewählt aus Polyamiden auf Kohlenwasserstoffbasis und Silikonpolyamiden und Mischungen davon, geliert ist; wobei die Phasen darin eine makroskopisch homogene Mischung bilden,

wobei die Zusammensetzung als hydrophile(s) Geliermittel/lipophile(s) Geliermittel-System ein System enthält, das ausgewählt ist aus:

- Copolymer von 2-Acrylamido-2-methylpropansulfonsäure und von Hydroxyethylacrylat/Kohlenwasserstoff-basiertem Polyamid; und
- Copolymer von 2-Acrylamido-2-methylpropansulfonsäure und von Hydroxyethylacrylat/Silikon-Polyamid.

**2.** Zusammensetzung nach vorangehendem Anspruch, die mindestens einen Farbstoff enthält, der vorzugsweise mindestens in der gelierten Ölphase vorliegt.

**3.** Zusammensetzung nach einem der vorangehenden Ansprüche, die als hydrophile(s) Geliermittel/lipophile(s) Geliermittel-System ein System enthält, ausgewählt aus:

- Copolymer von 2-Acrylamido-2-methylpropansulfonsäure und von Hydroxyethylacrylat/Ethylendiamin/Stearyldimer-Dilinoleat-Copolymer; und
- Copolymer von 2-Acrylamido-2-methylpropansulfonsäure und von Hydroxyethylacrylat/Nylon-611/Dimethicon-Copolymer.

**4.** Zusammensetzung nach einem der vorangehenden Ansprüche, enthaltend die wässrigen und öligen Phasen in einem Gewichtsverhältnis von 95/5 bis 5/95, vorzugsweise 30/70 bis 80/20, insbesondere 60/40 bis 80/20, vorzugsweise 60/40 bis 70/30 und insbesondere 60/40 bis 70/30.

**5.** Zusammensetzung nach einem der vorangehenden Ansprüche, die in Form einer Grundierung, eines Gesichtspuders, eines Lidschattens, eines Lippenstiftes, einer Mascara und/oder einer Pflegezusammensetzung vorliegt.

**6.** Zusammensetzung nach einem der vorangehenden Ansprüche, ferner umfassend feste Partikel wie Pigmente und/oder Füllstoffe und insbesondere 0,01 Gew.-% bis 25 Gew.-%, insbesondere 0,1 Gew.-% bis 25 Gew.-%, insbesondere 1 Gew.-% bis 20 Gew.-% und vorzugsweise 5 Gew.-% bis 15 Gew.-% feste Partikel bezogen auf das Gesamtgewicht der Zusammensetzung.

**7.** Zusammensetzung nach einem der vorangehenden Ansprüche, ferner umfassend flüchtige und/oder nichtflüchtige Silikonöle.

**8.** Zusammensetzung nach einem der vorangehenden Ansprüche, ferner umfassend eine Feuchtigkeitscreme und vorzugsweise Glycerin.

**9.** Verfahren zur Herstellung einer kosmetischen Zusammensetzung zum Schminken und/oder Pflegen von Keratinmaterialien, insbesondere der Haut und/oder der Lippen, umfassend mindestens einen Schritt des Mischens von:

- mindestens einer wässrigen Phase, die mit mindestens einem hydrophilen Geliermittel geliert ist; und
- mindestens einer öligen Phase, die mit mindestens einem wasserstoffbindenden Polymer geliert ist;

unter Bedingungen, die zum Erhalten einer makroskopisch homogenen Mischung geeignet sind, wobei das (die) hydrophile(n) Geliermittel/das (die) lipophile(n) Geliermittel aus dem folgenden System ausgewählt ist (sind):

- Copolymer von 2-Acrylamido-2-methylpropansulfonsäure und von Hydroxyethylacrylat/Kohlenwasserstoff-basiertem Polyamid; und
- Copolymer von 2-Acrylamido-2-methylpropansulfonsäure und von Hydroxyethylacrylat/Silikon-Polyamid.

**10.** Verfahren nach vorstehendem Anspruch, umfassend einen Schritt des Mischens von mindestens drei oder sogar

mehr gelierten Phasen.

**11.** Verfahren nach einem der Ansprüche 9 und 10, wobei das Mischen bei Raumtemperatur durchgeführt wird.

**12.** Kosmetisches Kit zum Schminken und/oder Pflegen von Keratinmaterialien, insbesondere der Haut und/oder der Lippen, umfassend, in getrennten Behältern, mindestens eine wässrige Phase, die mit mindestens einem hydrophilen Geliermittel geliert ist, und mindestens eine ölige Phase, die mit mindestens einem wasserstoffbindenden Polymer geliert ist, sowie eine Anleitung zur Verwendung der Rezepturmischungen, wobei das (die) hydrophile(n) Geliermittel/lipophile(n) Geliermittel-System ausgewählt ist (sind) aus:

- Copolymer von 2-Acrylamido-2-methylpropansulfonsäure und von Hydroxyethylacrylat/Kohlenwasserstoff-basiertem Polyamid; und
- Copolymer von 2-Acrylamido-2-methylpropansulfonsäure und von Hydroxyethylacrylat/Silikon-Polyamid.

**13.** Vorrichtung zum Schminken und/oder Pflegen von Keratinmaterialien, insbesondere der Haut und/oder der Lippen, umfassend mindestens:

- zwei getrennte Behälter, die jeweils eine wässrige Phase, die mit mindestens einem hydrophilen Geliermittel geliert ist, und eine ölige Phase, die mit mindestens einem Wasserstoffbindenden Polymer geliert ist, enthalten, wobei das (die) hydrophile(n) Geliermittel/lipophile(n) Geliermittel-System ausgewählt ist aus:
- Copolymer von 2-Acrylamido-2-methylpropansulfonsäure und von Hydroxyethylacrylat/Kohlenwasserstoff-basiertem Polyamid; und
- Copolymer von 2-Acrylamido-2-methylpropansulfonsäure und Hydroxyethylacrylat/Silikon-Polyamid;
- eine separate Kammer zum Mischen der Behälter, umfassend eine Öffnung, die konfiguriert ist, um das Einführen der zu mischenden Phasen zu ermöglichen; und
- ein Mittel zum Verteilen einer makroskopisch homogenen Mischung der beiden Phasen.

**14.** Kosmetisches Verfahren zum Schminken und/oder Pflegen von Keratinmaterialien, insbesondere der Haut und/oder der Lippen, umfassend mindestens einen Schritt, der darin besteht, auf das Keratinmaterial eine Zusammensetzung nach einem der Ansprüche 1 bis 8 aufzubringen.

**15.** Kosmetisches Verfahren zum Schminken und/oder Pflegen eines Keratinmaterials, insbesondere der Haut und/oder der Lippen, umfassend mindestens das Auftragen einer Zusammensetzung auf das Material, die durch unmittelbares Mischen mindestens einer wässrigen Phase, die mit mindestens einem hydrophilen Geliermittel geliert ist, vor der Anwendung oder zum Zeitpunkt der Anwendung auf das Keratinmaterial erhalten wurde, und mindestens einer öligen Phase, die mit mindestens einem wasserstoffbindenden Polymer geliert ist, wobei die Zusammensetzung als hydrophile(s) Geliermittel/lipophile(s) Geliermittel-System ein System enthält, das ausgewählt ist aus:

- Copolymer von 2-Acrylamido-2-methylpropansulfonsäure und von Hydroxyethylacrylat/Kohlenwasserstoff-basiertem Polyamid; und
- Copolymer von 2-Acrylamido-2-methylpropansulfonsäure und von Hydroxyethylacrylat/Silikon-Polyamid.

## Revendications

**1.** Composition cosmétique de maquillage et/ou de soin des matières kératiniques, en particulier de la peau et/ou des lèvres, comprenant :

- au moins une phase aqueuse gélifiée par au moins un agent gélifiant hydrophile choisi parmi les agents gélifiants polymères synthétiques ; et
- au moins une phase huileuse gélifiée par au moins un polymère à liaisons hydrogène choisi parmi les polyamides hydrocarbonés et les polyamides siliconés, ainsi que leurs mélanges ;

lesdites phases y formant un mélange macroscopiquement homogène,
laquelle composition contient, à titre de système de gélifiant(s) hydrophile(s) /gélifiant(s) lipophile(s), un système choisi parmi :

- un copolymère d'acide 2-acrylamido-2-méthylpropanesulfonique et d'acrylate d'hydroxyéthyle/un polyamide

hydrocarboné ; et
- un copolymère d'acide 2-acrylamido-2-méthylpropanesulfonique et d'acrylate d'hydroxyéthyle/un polyamide siliconé.

2. Composition selon la revendication précédente, contenant au moins une matière colorante, de préférence présente au moins dans la phase huileuse gélifiée.

3. Composition selon l'une quelconque des revendications précédentes, contenant, à titre de système de gélifiant(s) hydrophile(s) /gélifiant(s) lipophile(s), un système choisi parmi :

- un copolymère d'acide 2-acrylamido-2-méthylpropanesulfonique et d'acrylate d'hydroxyéthyle/un copolymère d'éthylènediamine et de dilinoléate dimère de stéaryle ; et
- un copolymère d'acide 2-acrylamido-2-méthylpropanesulfonique et d'acrylate d'hydroxyéthyle/un copolymère de Nylon-611 et de diméthicone.

4. Composition selon l'une quelconque des revendications précédentes, contenant les phases aqueuse et huileuse dans un rapport pondéral phase aqueuse/phase huileuse de 95/5 à 5/95 de préférence de 30/70 à 80/20, de préférence situé dans la plage allant de 60/40 à 80/20, de préférence situé dans la plage allant de 60/40 à 70/30, et mieux encore de 60/40 ou de 70/30.

5. Composition selon l'une quelconque des revendications précédentes, se présentant sous la forme d'un fond de teint, d'une poudre libre, d'un fard à paupières, d'un rouge à lèvres, d'un mascara et/ou d'une composition de soin.

6. Composition selon l'une quelconque des revendications précédentes, comprenant en outre des particules solides telles que des pigments et/ou des charges, et en particulier comprenant de 0,01 à 25 % en poids, tout spécialement de 0,1 à 25 % en poids, en particulier de 1 % à 20 % en poids et de préférence de 5 % à 15 % en poids de particules solides par rapport au poids total de ladite composition.

7. Composition selon l'une quelconque des revendications précédentes, comprenant en outre des huiles siliconées volatiles et/ou non volatiles.

8. Composition selon l'une quelconque des revendications précédentes, comprenant en outre un agent hydratant, de préférence le glycérol.

9. Procédé de préparation d'une composition cosmétique de maquillage et/ou de soin des matières kératiniques, en particulier de la peau et/ou des lèvres, comprenant au moins une étape de mélange :

- d'au moins une phase aqueuse gélifiée par au moins un agent gélifiant hydrophile ; et
- d'au moins une phase huileuse gélifiée par au moins un polymère à liaisons hydrogène ;

dans des conditions propices à l'obtention d'un mélange macroscopiquement homogène
le système de gélifiant(s) hydrophile(s)/gélifiant(s) lipophile(s) étant choisi parmi :

- un copolymère d'acide 2-acrylamido-2-méthylpropanesulfonique et d'acrylate d'hydroxyéthyle/un polyamide hydrocarboné ; et
- un copolymère d'acide 2-acrylamido-2-méthylpropanesulfonique et d'acrylate d'hydroxyéthyle/un polyamide siliconé.

10. Procédé selon la revendication précédente, comprenant une étape de mélange d'au moins trois phases gélifiées voire plus.

11. Procédé selon l'une quelconque des revendications 9 et 10, dans lequel le mélange est réalisé à température ambiante.

12. Kit cosmétique de maquillage et/ou de soin des matières kératiniques, en particulier de la peau et/ou des lèvres, comprenant, dans des contenants séparés, au moins une phase aqueuse gélifiée par au moins un agent gélifiant hydrophile, et au moins une phase huileuse gélifiée par au moins un polymère à liaisons hydrogène, ainsi qu'une notice de mise en oeuvre des mélanges extemporanés, le système de gélifiant(s) hydrophile(s)/gélifiant(s) lipophile(s) étant choisi parmi :

- un copolymère d'acide 2-acrylamido-2-méthylpropanesulfonique et d'acrylate d'hydroxyéthyle/un polyamide hydrocarboné ; et
- un copolymère d'acide 2-acrylamido-2-méthylpropanesulfonique et d'acrylate d'hydroxyéthyle/un polyamide siliconé.

**13.** Dispositif de maquillage et/ou de soin des matières kératiniques, notamment de la peau et/ou des lèvres, comportant au moins :

- deux contenants séparés contenant respectivement une phase aqueuse gélifiée par au moins un agent gélifiant hydrophile, et une phase huileuse gélifiée par au moins un polymère à liaisons hydrogène, le système de gélifiant(s) hydrophile(s)/gélifiant(s) lipophile(s) étant choisi parmi :
  - un copolymère d'acide 2-acrylamido-2-méthylpropanesulfonique et d'acrylate d'hydroxyéthyle/un polyamide hydrocarboné ; et
  - un copolymère d'acide 2-acrylamido-2-méthylpropanesulfonique et d'acrylate d'hydroxyéthyle/un polyamide siliconé ;
  - une chambre distincte pour mélanger lesdits contenants, comprenant une ouverture configurée pour permettre l'introduction desdites phases à mélanger ; et
  - un moyen de distribution d'un mélange macroscopiquement homogène des deux phases.

**14.** Procédé cosmétique de maquillage et/ou de soin de matières kératiniques, en particulier de la peau et/ou des lèvres, comprenant au moins une étape consistant à appliquer sur ladite matière kératinique une composition telle que définie selon l'une quelconque des revendications 1 à 8.

**15.** Procédé cosmétique de maquillage et/ou de soin d'une matière kératinique, en particulier de la peau et/ou des lèvres, comprenant au moins l'application sur ladite matière d'une composition obtenue par mélange extemporané, avant application ou au moment de l'application sur ladite matière kératinique, d'au moins une phase aqueuse gélifiée par au moins un agent gélifiant hydrophile et d'au moins une phase huileuse gélifiée par au moins un polymère à liaisons hydrogène, ladite composition contenant, à titre de système de gélifiant(s) hydrophile(s)/gélifiant(s) lipophile(s), un système choisi parmi :

- un copolymère d'acide 2-acrylamido-2-méthylpropanesulfonique et d'acrylate d'hydroxyéthyle/un polyamide hydrocarboné ; et
- un copolymère d'acide 2-acrylamido-2-méthylpropanesulfonique et d'acrylate d'hydroxyéthyle/un polyamide siliconé.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9965455 A
- WO 04057589 A
- WO 9962497 A
- JP 2005112834 A
- WO 2008081175 A
- EP 0815928 B1
- US 5981680 A
- WO 2008155059 A
- JP 09188830 A
- JP 10158450 A
- JP 10158541 A
- JP 07258460 A
- JP 05017710 A
- EP 1086683 A


### Non-patent literature cited in the description


- **ALMEIDA et al.** *Pharmaceutical Development and Technology,* 2008, vol. 13, 487
- Remington: The Science and Practice of Pharmacy. 1995, 282